# EUROPEAN PATENT SPECIFICATION

(11) **EP 3 033 151 B1**
(45) Date of publication and mention of the grant of the patent: **26.09.2018**
(21) Application number: 14739836.6
(22) Date of filing: 17.07.2014
(51) Int. Cl.: A61Q 11/00, A61Q 11/02, A61Q 17/00, A61K 8/27, A61K 8/24, A61K 8/02, A61K 8/11

(54) **ORAL CARE COMPOSITION**
MUNDPFLEGEMITTEL
COMPOSITION DE PROTECTION ORALE

(30) Priority: 16.08.2013 WO PCT/CN2013/081607; 17.09.2013 EP 13184665
(43) Date of publication of application: 22.06.2016
(73) Proprietor: Unilever N.V., 3013 AL Rotterdam (NL); Unilever PLC, London, Greater London EC4Y 0DY (GB)
(72) Inventor: DENG, Yan, Shanghai 201615 (CN); HOU, Wentao, Shanghai 201315 (CN); JIANG, Xue, Shanghai 312368 (CN); LI, Xiaoke, Shanghai 200335 (CN); LIU, Renjiang, Shanghai 200335 (CN)
(74) Representative: Tansley, Sally Elizabeth
(86) International application number: PCT/EP2014/065394
(87) International publication number: WO 2015/022141

(56) References cited:
- EP-A2- 0 428 493
- WO-A1-99/21547
- WO-A1-2006/012977
- WO-A1-2011/053291
- WO-A2-03/045344
- WO-A2-2011/056748
- DE-A1- 19 854 349
- GB-A- 1 241 402
- US-B1- 6 221 340

## Description

### TECHNICAL FIELD OF THE INVENTION

The present invention relates to oral care compositions such as tooth pastes, gums, mouthwashes and the like. In particular the present invention relates to oral care compositions containing a source of zinc ions and an orthophosphate source. The invention also relates to the use of such compositions for improving the oral hygiene of an individual.

### BACKGROUND TO THE INVENTION

Oral hygiene is a daily concern for most consumers. Unfortunately however, daily oral hygiene routines offer only limited protection against bacteria-mediated conditions and dental caries remains one of the most common diseases throughout the world.

To increase the antibacterial efficacy of oral care products, it is known to employ antibacterial metals such as zinc. Zinc can inhibit glycolysis and glucosyltransferases (GTFs) of bacterium even in a concentration as low as about 0.01 mM [Phan TN, Buckner T, Sheng J, et al., Oral Microbiology and Immunology, 2004, 19(1): 31-38]. Zinc has thus been formulated into oral-health products to give a range of benefits: It can reduce dental calculus formation, control plaque and reduce oral malodour.

Following dental hygiene practices such as tooth brushing, there is approximately 15-40% of total zinc in toothpaste that remains in the oral cavity but the zinc level in saliva and plaque falls rapidly over 30-60 minutes [Lynch, R. J. M., International Dental Journal. 61:46-54, 2011]. Thus efforts have been made to provide dentifrices having a longer lasting *in-situ* antibacterial benefit.

European patent application published as EP 0 539 651 A (SANGI CO, LTD) discloses a dentifrice having an antibacterial effect to prevent production of carious tooth and generation of periodontal diseases such as alveolar blennorrhea. The dentifrice contains hydroxylapatite powder. The hydroxylapatite powder carries therein an antibacterial metal such as silver, copper and/or zinc. The antibacterial metal is adsorbed to and/or combined, under ion exchange, with the hydroxylapatite powder. The antibacterial metal carried by the calcium compound is said to be highly stable such that the amount of released metal to water is extremely small, i.e., less than several ppb.

DE 198 54 349 A1 (UP TO DENT AG) discloses an agent for the care of the mouth containing chlorine dioxide sources and zinc salts in defined concentrations, useful for prevention of caries, gingivitis and parodontitis.

WO 03/045344 A2 (PROCTER & GAMBLE) discloses dentifrice compositions comprising linear polyphosphates, an ionic active ingredient and a binder system, wherein the dentifrice composition has a total water content of less than 10%.

WO 2006/012977 A1 (UNILEVER) discloses toothpaste composition and method for making such, the toothpaste comprising water, a zinc salt, a chelating agent for the zinc, the chelating agent having a log Ks1 as herein defined of from 3.0 to 7.0, and calcium carbonate as abrasive.

Unfortunately, by tightly combining the antibacterial metal with the hydroxyapatite, the metal ion may not necessarily be available to areas of the oral cavity (such as the soft tissue) away from the tooth surface and which may be reservoirs for caries-forming bacteria.

The present inventors have now recognized a need to provide an oral care composition which is capable of depositing zinc on the tooth surface and which can release at least some of the zinc into saliva.

### TESTS AND DEFINITIONS

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purposes of the present invention means a paste or gel dentifrice for use with a toothbrush. Especially preferred are tooth pastes suitable for cleaning teeth by brushing for about two minutes.

### Mouth Wash

"Mouth wash" for the purposes of the present invention means liquid dentifrice for use in rinsing the mouth. Especially preferred are mouth washes suitable for rinsing the mouth by swishing and/or gargling for about half a minute before expectorating.

### Solubility

Solubility as used herein refers to solubility of a source in pure water at 25 °C and 1 atm. Soluble refers to a source having a solubility of at least 0.1 mol dm⁻³. Insoluble refers to a source having a solubility of less than 0.1 mmol dm⁻³ (0.0001 mol dm⁻³). Sparingly soluble therefore refers to a source having a solubility of at least 0.1 mmol dm⁻³ but less than 0.1 mol dm⁻³.

### pH

When referring to the pH of an oral care composition, this means the pH measured when 1 part by weight of the composition is uniformly dispersed and/or dissolved in 20 parts by weight pure water at 25 °C. In particular the pH may be measured by manually mixing 1 g oral care composition with 20 ml water for 30 s, then immediately testing the pH with indicator paper or a pH meter. Where the composition is a dual-phase composition, the pH is determined immediately on combining the two phases, for example immediately upon dispensing the composition from its container.

### Substantially Free

"Substantially free of', as used herein, means less than 1.5%, and preferably less than 1.0%, and more preferably less than 0.75% and more preferably still less than 0.5% and most preferably from 0 to 0.1 % by weight, based on total weight of the oral care composition or phase referred to.

### Viscosity

Viscosity of a tooth paste is the value taken at room temperature (25 °C) with a Brookfield Viscometer, Spindle No. 4 and at a speed of 5 rpm. Values are quoted in centipoises (cP = mPa.s) unless otherwise specified.

### Particle Size

"Particle size" as used herein means diameter size and is reported as a weight average particle size. "Diameter" is meant to mean the longest length measurable in any dimension in the event the particle is not a perfect sphere. Particle size can be measured, for example by dynamic light scattering (DLS).

### In Situ

*"In situ"* as used herein means during and/or after application of the oral care composition to the oral cavity.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified.

It should be noted that in specifying any range of values, any particular upper value can be associated with any particular lower value.

References herein to salts (such as, for example, zinc citrate, zinc lactate, sodium hydrogen phosphate etc.) should be taken to mean the salt in any form including, for example, hydrate form.

For the avoidance of doubt, the word "comprising" is intended to mean "including" but not necessarily "consisting of' or "composed of'. In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### SUMMARY OF THE INVENTION

In a first aspect, the present invention is directed to an oral care composition comprising:
a) source of zinc; and
b) orthophosphate source;
wherein the molar ratio of zinc to orthophosphate (Zn:PO₄) in the composition is less than 1:1;
wherein if the oral care composition comprises a calcium source then the molar ratio of zinc to calcium (Zn:Ca) is at least 1:5;
wherein the pH of the composition is greater than 6.0;
wherein the pH of the composition is measured when 1 part by weight of the composition is uniformly dispersed and/or dissolved in 20 parts by weight pure water at 25°C; and wherein the composition is:
I) a mono-phase composition comprising the source of zinc and the orthophosphate source, wherein the composition comprises water in an amount of from 0 to 5% by weight of the composition;
II) a hydrous mono-phase composition wherein the source of zinc is encapsulated, the orthophosphate source is encapsulated, or both; or
III) a dual-phase composition comprising:
   (i) a first phase comprising the source of zinc; and
   (ii) a second phase comprising the orthophosphate source.

In a second aspect, the invention is directed to a method of improving the oral hygiene of an individual comprising applying the composition of any embodiment of the first aspect to at least one surface of the teeth of the individual. The method is preferably non-therapeutic. Alternatively, the method may be said to be use of the composition in the manufacture of a medicament for improving oral hygiene and/or to be the composition for use as a medicament, preferably for use as a medicament for improving oral hygiene.

All other aspects of the present invention will more readily become apparent upon considering the detailed description and examples which follow.

### BRIEF DESCRIPTION OF THE FIGURES

Certain aspects or embodiments of the present invention will be described by reference to the Figures, in which:
Fig. 1 shows FTIR spectra of reaction products formed in zinc citrate slurries in pH 8.0 sodium phosphate buffer and long reaction time where the Zn:PO₄ ratio was (a) 3:1, (b) 2:1, (c) 1:1, (d) 1:2, (e) 1:3 or (f) 1:4. Also shown are spectra of (g) zinc citrate and (h) Zn₃(PO₄)₂·4H₂O.
Fig. 2 shows FTIR spectra of reaction products formed in potassium phosphate / zinc sulphate slurries at long reaction time where the Zn:PO₄ ratio was (a) 2:1, (b) 1:1, (c) 1:2, (d) 1:3, (e) 1:4 or (f) 1:5.
Fig. 3 shows SEM images of bovine enamel blocks after brushing with zinc citrate slurried in sodium phosphate buffer having pH of (a) 6.0, (b) 7.0, (c) 8.0 or (d) 9.0. The scale bar is 5 micron.
Fig. 4 shows SEM images of bovine enamel blocks after brushing with zinc citrate slurried in potassium phosphate buffer having pH of (a) 6.0 or (b) 7.0. The scale bar is 10 micron.
Fig. 5 shows FTIR spectra of reaction products formed during brushing of the enamel blocks of Fig. 3 where the pH of the phosphate buffer was (b) 6.0, (c) 7.0, (d) 8.0 or (e) 9.0. Also shown are spectra of (a) zinc citrate and (f) Zn₃(PO₄)₂·4H₂O.
Fig. 6 shows FTIR spectra of reaction products formed in zinc citrate slurried in sodium phosphate buffer at long reaction time where the pH of the buffer was (b) 6.0, (c) 7.0, (d) 8.0 or (e) 9.0. Also shown are spectra of (a) zinc citrate and (f) Zn₃(PO₄)₂·4H₂O.
Fig. 7 shows FTIR spectra of reaction products formed during brushing of the enamel blocks of Fig. 4 where the pH of the phosphate buffer was (a) 4.0, (b) 5.0, (c) 6.0, (d) 7.0, (e) 8.0, (f) 9.0 or (g) 10.0. Also shown is the spectrum of (h) Zn₃(PO₄)₂·4H₂O.

### DETAILED DESCRIPTION

The only limitation with respect to the source of zinc for use in the present invention is that the same is suitable for oral use. Specific example of zinc sources include zinc chloride, zinc acetate, zinc ascorbate, zinc sulphate, zinc nitrate, zinc oxide, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate or a mixture thereof.

Without wishing to be bound by theory, the present inventors believe that the zinc source has the ability to react with phosphate ions to produce a zinc-containing phosphate salt *in situ* even when in normal use as part of a dentifrice and without any special procedures or implements. Such a zinc-containing phosphate salt may be capable of strongly adhering to tooth surfaces while allowing slow release of zinc into the oral cavity, so providing long-lasting and wide-ranging antibacterial action. Furthermore, the zinc source may allow for effective deposition of zinc onto teeth without the zinc being rapidly washed away and/or swallowed.

To minimize negative sensory properties of zinc ions (such as astringency) it is preferred that the zinc source is sparingly soluble. More preferably the solubility of the zinc source is less than 0.07 mol dm⁻³, more preferably less than 0.05 mol dm⁻³ and most preferably less than 0.03 mol dm⁻³. On the other hand, to allow the zinc source to release some zinc ions to be available for reaction with phosphate it is preferred that the solubility is not too low. Preferably the solubility of the zinc source is at least 0.5 mmol dm⁻³, more preferably at least 1 mmol dm⁻³ and most preferably at least 2 mmol dm⁻³.

Illustrative examples of the most preferred types of zinc source that may be used in this invention include, for example zinc citrate, zinc lactate or a mixture thereof. Most preferably the zinc source comprises or is zinc citrate.

In an alternative embodiment, the zinc source may be soluble. Soluble sources may be able to react more quickly with phosphate ions leading to more immediate effects. Specific examples of soluble zinc sources include, for example, zinc chloride, zinc acetate, zinc ascorbate, zinc sulphate, zinc nitrate, or mixture thereof.

Preferably the zinc source is present in the composition such that the total amount of zinc in the composition is from 0.01 to 10% by weight of the composition. More preferably the composition comprises from 0.05 to 5% and most preferably from 0.1 to 2% zinc by weight of the composition.

To maximise deposition efficiency, especially where the zinc source is sparingly soluble, it is preferred that the zinc source is particulate. More preferably the zinc source is present in the composition with a particle size of less than 50 micron, more preferably from 1 to 20 micron, most preferably from 0.1 to 1 micron.

The present inventors have found that the presence of large amounts of calcium in the composition can inhibit deposition of zinc. Without wishing to be bound by theory the present inventors believe this may be due to the formation of calcium phosphate salts which deposit on the tooth surface to inhibit deposition of zinc-containing salts. Thus if the oral care composition comprises a calcium source then the molar ratio of zinc to calcium (Zn:Ca) is at least 1:5, preferably at least 1:2, more preferably at least 1:1, more preferably still at least 2:1, even more preferably at least 5:1 and most preferably in the range 10:1 to 1000:1. In an especially preferred embodiment the composition is substantially free of calcium, most preferably the composition is substantially free from multi-valent metal ions other than zinc.

The orthophosphate source for use in the present invention is preferably soluble. Without wishing to be bound by theory, the present inventors believe that the presence of a soluble phosphate source allows for the phosphate ions to react with the zinc source to produce a zinc-containing phosphate salt *in situ* even when in normal use as part of a dentifrice and without any special procedures or implements.

Suitable orthophosphate sources include one or more salts of formula (I):

M₍₃₋ₙ₎HₙPO₄ (I);

wherein:
- M is potassium, sodium, ammonium or a combination thereof;
- n is 0, 1 or 2.

Most preferred are salts according to formula (I) wherein M is potassium and/or sodium. Specific examples include sodium dihydrogen phosphate, disodium hydrogen phosphate, trisodium phosphate, potassium dihydrogen phosphate, dipotassium hydrogen phosphate, tripotassium phosphate, or a mixture thereof. In a most preferred embodiment, the orthophosphate source used is trisodium phosphate and monosodium dihydrogen phosphate at a trisodium phosphate to monosodium dihydrogen phosphate weight ratio of 1:4 to 4:1, more preferably 1:3 to 3:1, and most preferably, from 1:2 to 2:1.

Preferably the orthophosphate source is present in the composition such that the total amount of orthophosphate (i.e., PO₄) in the composition is from 0.05 to 20% by weight of the composition. More preferably the composition comprises from 0.1 to 15% and most preferably from 1 to 10% orthophosphate by weight of the composition.

The present inventors have found that at relatively acidic pH values, the zinc and orthophosphate sources have a tendency to react *in situ* such the reaction product is mainly composed of Zn₃(PO₄)₂. Unfortunately Zn₃(PO₄)₂ has very low solubility in oral fluid and so does not release desirable quantities of zinc ions. On the other hand zinc phosphate salts which also contain monovalent cations have higher solubility in saliva. Thus it is preferred that the composition is formulated such that the source of zinc and orthophosphate source react *in situ* to form a reaction product comprising one or more monovalent cations, especially sodium, potassium or a combination thereof.

The present inventors have found that formation of salts other than Zn₃(PO₄)₂ is favoured at pH values greater than 6.0. Thus the composition has a pH of greater than 6.0. More preferably the pH of the composition is at least 6.2, more preferably still at least 6.5, even more preferably at least 6.7 and most preferably at least 7.0. To avoid unpleasant sensory properties it is preferred that the pH is not too high. Preferably the pH of the composition is less than 11, more preferably less than 10.5, more preferably still less than 9.5 and most preferably less than 9.0.

The molar ratio of zinc to orthophosphate (Zn:PO₄) in the composition is less than 1:1. The present inventors have found that at high ratios of Zn:PO₄ the zinc and orthophosphate sources do not tend to react *in-situ* to form zinc phosphate salts.

The ratio of Zn:PO₄ is however preferably not too low in order to prevent excessive formation of Zn₃(PO4)₂. Thus it is preferred that the ratio of Zn:PO₄ is at least 1:20, more preferably at least 1:10, more preferably at least 1:5, and most preferably at least 1:2.

The oral care composition is preferably provided in a format wherein premature reaction of the zinc and orthophosphate sources is avoided. This may be achieved in a number of ways but preferred embodiments include dual-phase compositions and/or anhydrous compositions. As used herein "anhydrous" refers to compositions which comprise less than 5% water by weight of the composition and preferably which are substantially free of water. Therefore "hydrous" refers to compositions which comprise at least 5% water by weight of the composition, more preferably from 20 to 90%.

Dual-phase compositions comprise:
(i) a first phase comprising the source of zinc; and
(ii) a second phase comprising the orthophosphate source.

Typically the first phase will be substantially free from orthophosphate. Preferably also the second phase is substantially free from zinc. The first and / or second phases are preferably hydrous and comprise at least 5% water by weight of the phase, more preferably from 20 to 90%. The two phases are preferably stored in a single container (such as, for example, a tube) but are physically separated within the container (for example, by being present in separate compartments within the container). Such a container will typically be equipped with means for simultaneously delivering portions of each phase to the surface of a tooth, for example by co-extrusion of the phases through an orifice.

In another embodiment the oral care composition is a mono-phase composition comprising the source of zinc and the orthophosphate source in the same phase. In such a composition, the zinc and orthophosphate sources are inhibited from prematurely reacting owing to the absence of suitable solvent (i.e. water).

Dual-phase formats are especially preferred where the zinc source is soluble.

Another strategy for separating the zinc and phosphate sources which may allow for alternative formats (such as hydrous mono-phase compositions) is, for example, encapsulation of one or both sources.

The composition of the present invention preferably comprises physiologically acceptable carrier in addition to the zinc and orthophosphate sources. Typically the carrier comprises at least surfactant, thickener, humectant or a combination thereof.

Preferably the composition comprises a surfactant. Preferably the composition comprises at least 0.01 % surfactant by weight of the oral care composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum Arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, sodium carboxymethyl cellulose and/or a Carbomer is/are preferred. When a Carbomer is employed, those having a molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickener typically makes up from 0.01 to about 10%, and preferably, from 0.1 to 8%, and most preferably, from 1.5 to 6% by weight of the oral care composition, based on total weight of the composition and including all ranges subsumed therein.

When the dentifrice composition of this invention is a toothpaste, the same typically has a viscosity from about 50,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. Preferably, the humectant makes up from 25 to 80%, and most preferably, from 45 to 70% by weight of the oral care composition, based on total weight of the composition and including all ranges subsumed therein.

Oral care compositions described herein may comprise optional ingredients which are common in the art. These ingredients include antimicrobial agents, anti-inflammatory agents, anti-caries agents, plaque buffers, vitamins, fluoride sources, plant extracts, desensitizing agents, anti-calculus agents, biomolecules, flavors, proteinaceous materials, preservatives, opacifying agents (especially titanium dioxide), coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the oral care composition described herein, and preferably, from 0.0 to 15% by weight, and most preferably, from 0.01 to 12% by weight of the composition, including all ranges subsumed therein.

The oral care composition can be used in a method of improving the oral hygiene of an individual comprising applying the composition to at least one surface of the teeth of the individual. The oral care composition of this invention may additionally or alternatively be used in the manufacture of a medicament for providing an oral care benefit as described herein, such as for example, for the treatment or prevention of dental caries.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 10 s to 10 hours, more preferably still from 30 s to 1 hour and most preferably from 1 minute to 5 minutes. The composition may be used daily, for example for use by an individual once, twice or three times per day.

The following examples are provided to facilitate an understanding of the present invention. The examples are not provided to limit the scope of the claims.

### EXAMPLES

### Example 1

This example demonstrates the effect of calcium on deposition of zinc on simulated tooth surfaces (hydroxyapatite discs).

6 mm diameter hydroxyapatite discs were polished by using fine sand paper (1500) for 2 mins, to ensure smooth surfaces.

Simple toothpaste systems were prepared with the compositions shown in Table 1.

**TABLE 1**

| **Component % w/w** | **Sample A** | **Sample B** |
|---|---|---|
| Glycerine | To 100 | To 100 |
| Calcium Silicate* | 15 | --- |
| Na₃PO₄ | 3.82 | 3.82 |
| NaH₂PO₄ | 3.21 | 3.21 |
| Zinc Citrate Trihydrate** | 2.00 | 2.00 |

| | | |
|---|---|---|
| *PQ Brand Calcium Silicate with CaO:SiO₂ molar ratio of 1:3.66. **(C₆H₅O₇)₂Zn₃.3H₂O | | |

The molar ratio Zn:PO₄ in these samples is 1:5.2. The molar ratio of Zn:Ca in sample A is 1:5.7.

Portions of each sample were slurried with deionised water in a weight ratio of 2:1 (water: sample). The HAP discs were then immediately brushed with a slurry for 1 minute using an automated process. After soaking in the slurry for a further 1 min the disks were rinsed twice with de-ionized water. For certain discs the brushing process was repeated to a total of 14 times, while others were brushed for a total of 46 times.

The zinc which had deposited on the discs was then quantified using ICP-OES (inductively coupled plasma optical emission spectrometry). To prepare the treated discs for analysis, all surfaces except for the top (brushed) surfaces were coated with nail varnish (a separate experiment indicated nail varnish is not soluble in 10% HNO₃ solution) to exclude the zinc from these surfaces being extracted. The discs were then subjected to three times extraction by soaking 1 ml of 10% HNO₃ for 10 minutes at 37 °C and three times of rinsing with the same HNO₃ solution.

The amount of zinc extracted by the HNO₃ and the known surface area of the discs was used to calculate the amount of zinc deposited in µg per cm². The results are shown in Table 2 wherein the values are the mean of 4 measurements and the error quoted is the 95% confidence interval.

**TABLE 2**

| **Sample** | **Zinc Deposition after 14 brushings** | **Zinc Deposition after 46 brushings** |
|---|---|---|
| A | 105 ± 19 | 106 ± 23 |
| B | 146 ± 12 | 661 ± 48 |

These results illustrate that the sample which contained a large amount of calcium (Sample A) had inferior zinc deposition compared to that of the sample that contained no calcium (Sample B).

### Example 2

This example demonstrates the effect of the ratio of zinc to orthophosphate on the composition of reaction products.

Phosphate buffer (0.23 M) with pH 8.0 was prepared by dissolving 2.8299 g anhydrous trisodium phosphate and 2.0710 g anhydrous disodium hydrogen phosphate in 150 ml deionized water.

Reaction slurries were then prepared by adding 0.576 g, 0.385 g, 0.193 g, 0.096 g, 0.064 g or 0.048 g zinc citrate trihydrate to 4 ml portions of the phosphate buffer. Each slurry was then diluted by adding 46 ml deionized water to yield slurries with final molar ratios of Zn:PO₄ of 3:1, 2:1, 1:1, 1:2, 1:3 and 1:4. These slurries were shaken overnight and then centrifuged at 10000 rpm for 3 min. The supernatant was removed and the solids washed twice, using 10 ml deionized water each time. The samples were centrifuged again to remove the water and dried in an oven at 37 °C for 5 hours.

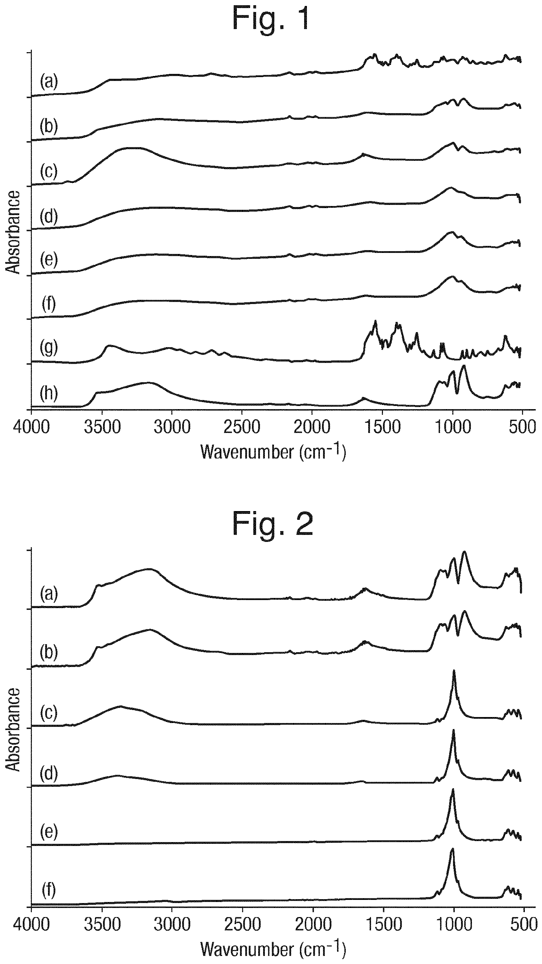

The infra-red spectrum of the precipitate from each reaction slurry was recorded using a Nicolet 6700 FTIR spectrometer with ATR diamond accessory and the spectra are shown in Fig. 1.

As can be seen from this figure, the reaction products formed at a Zn:PO₄ molar ratio of 3:1 (Fig. 1a) are similar to zinc citrate without any phosphate present (Fig. 1g). In contrast, the products formed at Zn:PO₄ molar ratio of 2:1 and below (Figs. 1b-f) each had a spectrum quite different to that of zinc citrate or that of Zn₃(PO₄)₂ (Fig. 1h). These data indicate that at ratios of Zn:PO₄ above 2:1, even after long reaction times a large proportion of the zinc citrate does not react to form zinc phosphate salts and at ratios of 2:1 and below, zinc phosphate salts other than Zn₃(PO₄)₂ are formed.

### Example 3

This example demonstrates the effect of the ratio of zinc to orthophosphate on the composition of reaction products.

Phosphate buffer (0.23 M) with pH about 9 was prepared by dissolving 26.2453g K₂HPO₄.3H₂O in deionized water and made up to 500 ml.

Reaction slurries were then prepared by adding 0.1329 g, 0.1660 g, 0.2218 g, 0.3315 g, 0.6620 g or 1.3230 g ZnSO₄.7H₂O to 10 ml portions of the phosphate buffer (molar Zn:PO₄ ratios were 1:5, 1:4, 1:3, 1:2, 1:1 and 2:1, respectively). These slurries were shaken overnight and then centrifuged at 10000 rpm for 3 min. The supernatant was removed and the solids washed twice, using 10 ml deionized water each time. The samples were centrifuged again to remove the water and dried in an oven at 37 °C for 5 hours.

The infra-red spectrum of the precipitate from each reaction slurry was recorded using a Nicolet 6700 FTIR spectrometer with ATR diamond accessory and the spectra are shown in Fig. 2.

As can be seen from this figure, the reaction products formed at a Zn:PO₄ molar ratio of 2:1 (Fig. 2a) and 1:1 (Fig. 2b) had spectra similar to that of Zn₃(PO₄)₂ (Fig. 1h). In contrast, the products formed at lower molar Zn:PO₄ ratios of 1:2, 1:3, 1:4 and 1:5 (Figs 2c-f) each had a spectrum quite different to that of Zn₃(PO₄)₂, indicating that the reaction products incorporate ions other than zinc and orthophosphate.

This example demonstrates that lower molar ratios of Zn:PO₄ generally favour formation of phosphate salts other than Zn₃(PO₄)₂.

### Example 4

This example demonstrates the effect of pH on deposition and composition of reaction products of a sodium orthophosphate and zinc citrate.

Phosphate solutions were prepared by titrating a 0.15 M NaH₂PO₄ solution to pH 6.0, 7.0, 8.0 and 9.0 using 2.5 M NaOH solution.

Polished bovine enamel blocks were then treated with 0.06 g zinc citrate trihydrate slurried in 10 ml of each phosphate solution. The treatment involved machine brushing the blocks for 1 min, leaving the blocks immersed in slurry for a further 1 min, and then rinsing the blocks twice (15 ml de-ionized water for each rinse).

The rinsed bovine enamel blocks were allowed to dry at room temperature (25 °C) and characterized by SEM.

Each slurry was also collected and centrifuged and the resulting precipitates washed with 25 ml de-ionized water for 3 times before drying at room temperature. The infra-red spectrum of each precipitate was recorded using a Nicolet 6700 FTIR spectrometer with ATR diamond accessory.

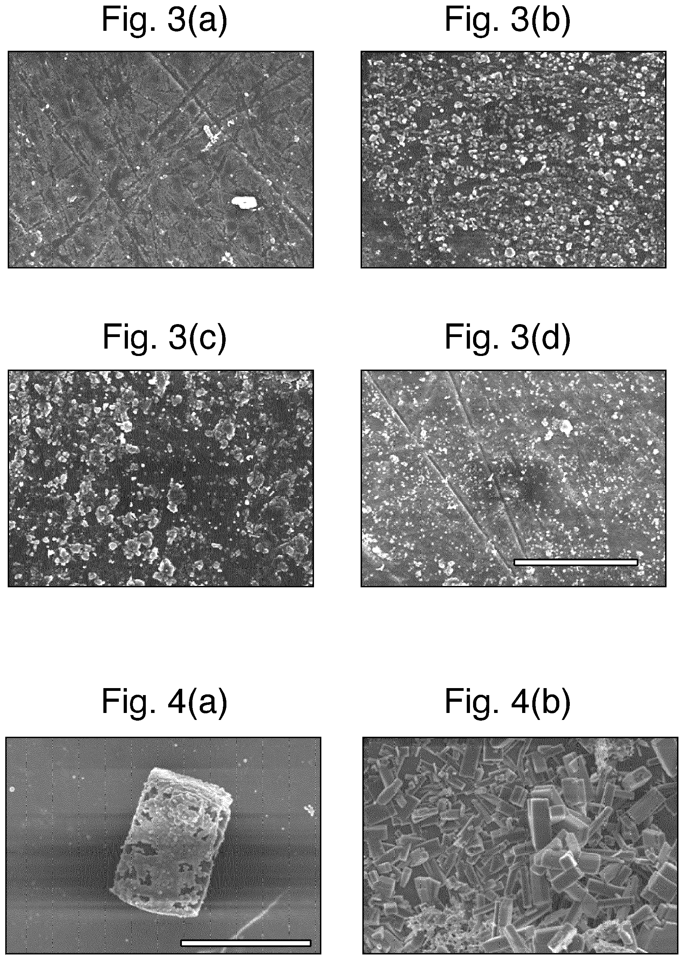

Representative SEM micrographs of the treated surface of the blocks are shown in Fig. 3. From this it can be seen that there are hardly any particles deposited on the surface of the block treated using phosphate buffer at pH 6.0 (Fig. 3a). In contrast, the blocks treated with phosphate buffers at pH 7.0 (Fig. 3b), 8.0 (Fig. 3c) or 9.0 (Fig. 3d) each show a large number of particles deposited on the surface.

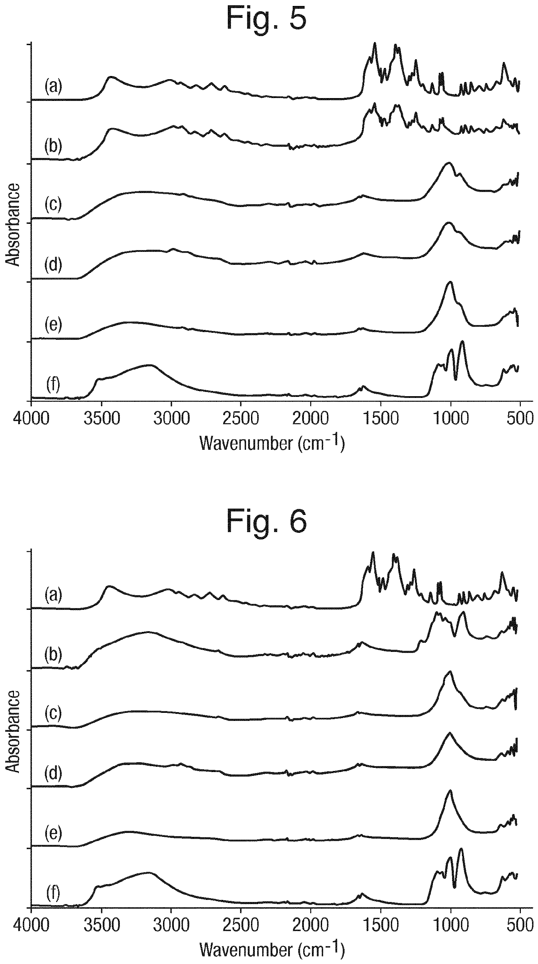

The FTIR spectra shown in Fig. 5 demonstrate that the reaction products formed from phosphate buffer at pH 6.0 (Fig. 5b) were largely similar to zinc citrate (Fig. 5a), indicating that little transformation to zinc phosphate salts occurred over the 2 min brushing period. In contrast, the products formed from phosphate buffer at pH 7.0 (Fig. 5c), 8.0 (Fig. 5d) or 9.0 (Fig. 5e) each had a spectrum closer to that of Zn₃(PO₄)₂ (Fig. 3f). These spectra were not, however, identical to that of Zn₃(PO₄)₂, indicating that other ions (e.g. Na⁺) had been incorporated into the reaction products. This was also confirmed by elemental analysis (using EDX = energy dispersion X-ray spectroscopy) which showed that significant amounts of sodium were incorporated in the precipitates.

Further slurries were prepared in an identical manner as described above except instead of brushing for 2 min, the slurries were stirred overnight (approximately 18 hours) before separation and drying of the precipitates.

The FTIR spectra of these precipitates are shown in Fig. 6. As can be seen from this figure, the reaction products formed from phosphate buffer at pH 6.0 (Fig. 6b) are similar to Zn₃(PO₄)₂ (Fig. 6f). In contrast, the products formed from phosphate buffer at pH 7.0 (Fig. 6c), 8.0 (Fig. 6d) or 9.0 (Fig. 6e) each had a spectrum quite different to that of Zn₃(PO₄)₂ (Fig. 6f). These data again indicate that using phosphate buffer at pH values above 6.0, the reaction products incorporate ions other than zinc and orthophosphate.

### Example 5

This example demonstrates the effect of pH on deposition and composition of reaction products of a potassium orthophosphate and zinc citrate.

Phosphate solutions were prepared by titrating a 0.15 M KH₂PO₄ solution to pH 4.0, 5.0, 6.0, 7.0, 8.0, 9.0 and 10.0 using 2.5 M NaOH solution or 10% nitric acid solution.

Bovine enamel blocks were brushed with slurries of zinc citrate and the phosphate solutions in an identical manner as described in Example 4.

Representative SEM micrographs of the treated surface of some of the blocks are shown in Fig. 4. With phosphate buffers at pH values of 6.0 and below (see Fig. 4a for example) only a few, isolated particles were deposited and these had the size and shape of the zinc citrate raw material. In contrast, the blocks treated with phosphate buffer at pH 7.0 and above (see Fig. 4b for example) each had a mass of small, cuboid particles covering the entire surface.

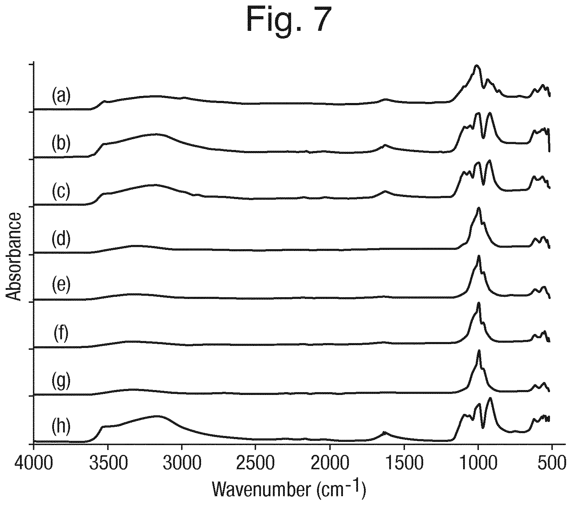

Fig. 7 shows the FTIR spectra of the precipitates collected from the slurries after brushing. The reaction products formed from phosphate buffers at pH 6.0 and below (Figs. 7a-c) were most similar to Zn₃(PO₄)₂ (Fig. 7h). In contrast, the products formed from phosphate buffer at pH 7.0 or above (Figs. 7d-g) each had a spectrum quite different to that of Zn₃(PO₄)₂, indicating that with phosphate buffer at pH values above 6.0, the reaction products incorporate ions other than zinc and orthophosphate. This was also confirmed by elemental analysis (using EDX) which showed that significant amounts of potassium were incorporated in the precipitates.

### Example 6

An example anhydrous monophase toothpaste formulation according to the invention is given in Table 3.

**TABLE 3**

| **Component** | **% w/w** |
|---|---|
| Glycerine | To 100 |
| PEG 400 | 10.50 |
| Flavour | 1.20 |
| Trisodium Phosphate | 4.36 |
| Zinc Citrate Trihydrate | 2.00 |
| Sodium Monofluorophosphate | 1.11 |
| Sweetener | 0.20 |
| Monosodium Phosphate | 3.20 |
| PEG 3000 | 1.75 |
| Pigment | 0.05 |
| Abrasive Silica | 7.00 |
| Sodium Lauryl Sulphate | 2.00 |

### Example 7

An example dual-phase toothpaste formulation according to the invention is given in Table 4, wherein the phases can be combined in a weight ratio of about 1:1 immediately prior to use.

**TABLE 4**

| **Dual-phase toothpaste** | | | |
|---|---|---|---|
| **Zinc Phase** | | **Phosphate Phase** | |
| **Component** | **wt%** | **Component** | **wt%** |
| Chlorinated water | To 100 | Chlorinated Water | To 100 |
| Sweetener | 0.20 | PEG1500 | 2.000 |
| Potassium Nitrate | 0.50 | Sweetener | 0.200 |
| Sorbitol(70%) | 50.00 | NaH₂PO₄ | 5.620 |
| Benz. Alcohol | 0.50 | Na₃PO₄ | 8.720 |
| Zinc Citrate Trihydrate | 4.00 | Sorbitol(70%) | 55.000 |
| Abrasive silica | 8.00 | Silica Abrasive | 8.000 |
| SCMC | 0.50 | Thickening Silica | 5.500 |
| Sodium Monofluorophosphate | 1.11 | Sodium Lauryl Sulphate | 2.200 |
| Flavour | 0.90 | Flavour | 1.200 |
| Sodium Lauryl Sulphate | 2.20 | SCMC | 0.500 |
| Potassium Citrate | 0.50 | Sodium Monofluorophosphate | 1.110 |
| Thickening Silica | 8.50 | Colour | 0.002 |

## Claims

1. An oral care composition comprising:
a) source of zinc; and
b) orthophosphate source;
wherein the molar ratio of zinc to orthophosphate (Zn:PO₄) in the composition is less than 1:1;
wherein if the oral care composition comprises a calcium source then the molar ratio of zinc to calcium (Zn:Ca) is at least 1:5;
wherein the pH of the composition is greater than 6.0;
wherein the pH of the composition is measured when 1 part by weight of the composition is uniformly dispersed and/or dissolved in 20 parts by weight pure water at 25°C; and
wherein the composition is:
I) a mono-phase composition comprising the source of zinc and the orthophosphate source, wherein the composition comprises water in an amount of from 0 to 5% by weight of the composition;
II) a hydrous mono-phase composition wherein the source of zinc is encapsulated, the orthophosphate source is encapsulated, or both; or
III) a dual-phase composition comprising:
(i) a first phase comprising the source of zinc; and
(ii) a second phase comprising the orthophosphate source.

2. The oral care composition as claimed in claim 1, wherein the ratio of Zn:PO₄ is in the range of less than 1:1 to 1:20.

3. The oral care composition as claimed in claim 1 or claim 2, wherein the composition is a mono-phase composition comprising water in an amount of from 0 to 5% by weight of the composition.

4. The oral care composition as claimed in any one of the preceding claims, wherein the pH of the composition is at least 6.5.

5. The oral care composition as claimed in any one of the previous claims wherein the source of zinc is sparingly soluble having a solubility of at least 0.1 mmol dm⁻³ but less than 0.1 mol dm⁻³, preferably a sparingly-soluble source of zinc comprising zinc citrate, zinc lactate or a mixture thereof.

6. The oral care composition as claimed in any one of claims 1 to 4 wherein the source of zinc is soluble having a solubility of at least 0.1 mol dm⁻³, preferably a soluble source of zinc comprising zinc chloride, zinc acetate, zinc ascorbate, zinc sulphate, zinc nitrate, or mixture thereof.

7. The oral care composition as claimed in any one of the previous claims wherein the orthophosphate source is soluble having a solubility of at least 0.1 mol dm⁻³.

8. The oral care composition as claimed in claim 7 wherein the orthophosphate comprises one or more salts of formula (I):
M₍₃₋ₙ₎HₙPO₄ (I);
wherein:
- M is potassium, sodium, ammonium or a combination thereof;
- n is 0, 1 or 2.

9. The oral care composition as claimed in any preceding claim wherein the composition is a dentifrice, preferably a toothpaste.

10. The oral care composition as claimed in any one of the preceding claims, wherein the composition comprises a surfactant.

11. The oral care composition as claimed in any one of the preceding claims wherein the total amount of zinc in the composition is from 0.01 to 10% by weight of the composition.

12. The oral care composition as claimed in any one of the preceding claims, wherein the total amount of orthophosphate (PO₄) in the composition is from 0.05 to 20% by weight of the composition.

13. An oral care composition as claimed in any one of the preceding claims for use in improving the oral hygiene of an individual comprising applying the composition to at least one surface of the teeth of the individual.

## Patentansprüche

1. Mundpflegezusammensetzung, umfassend:
a) Zink-Quelle und
b) Orthophosphat-Quelle,
wobei das Molverhältnis von Zink zum Orthophosphat (Zn:PO₄) in der Zusammensetzung weniger als 1:1 beträgt,
wobei dann, wenn die Mundpflegezusammensetzung eine Calcium-Quelle umfasst, das Molverhältnis von Zink zu Calcium (Zn:Ca) mindestens 1:5 beträgt,
wobei der pH der Zusammensetzung größer als 6,0 ist,
wobei der pH der Zusammensetzung gemessen wird, wenn 1 Gewichtsteil der Zusammensetzung bei 25°C in 20 Gewichtsteilen reinen Wassers gleichmäßig dispergiert und/oder aufgelöst wird, und
wobei die Zusammensetzung darstellt:
I) eine einphasige Zusammensetzung, die die Zink-Quelle und die Orthophosphat-Quelle umfasst, wobei die Zusammensetzung Wasser in einer Menge von 0 bis 5 Gewichts-% der Zusammensetzung umfasst,
II) eine wässrige einphasige Zusammensetzung, wobei die Zink-Quelle eingekapselt ist, die Orthophosphat-Quelle eingekapselt ist oder beide sind oder
III) eine zweiphasige Zusammensetzung, umfassend:
(i) eine erste Phase, umfassend die Zink-Quelle, und
(ii) eine zweite Phase, umfassend die Orthophosphat-Quelle.

2. Mundpflegezusammensetzung, wie im Anspruch 1 beansprucht, wobei das Verhältnis von Zn:PO₄ in dem Bereich von weniger als 1:1 bis 1:20 liegt.

3. Mundpflegezusammensetzung, wie im Anspruch 1 oder Anspruch 2 beansprucht, wobei die Zusammensetzung eine einphasige Zusammensetzung ist, die Wasser in einer Menge von 0 bis 5 Gewichts-% der Zusammensetzung umfasst.

4. Mundpflegezusammensetzung, wie in irgendeinem der vorhergehenden Ansprüche beansprucht, wobei der pH der Zusammensetzung mindestens 6,5 beträgt.

5. Mundpflegezusammensetzung, wie in irgendeinem der vorhergehenden Ansprüche beansprucht, wobei die Zink-Quelle schwerlöslich ist und eine Löslichkeit von mindestens 0,1 mMol dm⁻³, jedoch weniger als 0,1 Mol dm⁻³ aufweist, vorzugsweise eine schwerlösliche Zink-Quelle ist, die Zinkcitrat, Zinklactat oder eine Mischung davon umfasst.

6. Mundpflegezusammensetzung, wie in irgendeinem der Ansprüche 1 bis 4 beansprucht, wobei die Zink-Quelle löslich ist und eine Löslichkeit von mindestens 0,1 Mol dm⁻³ aufweist, vorzugsweise eine lösliche Zink-Quelle ist, die Zinkchlorid, Zinkacetat, Zinkascorbat, Zinksulfat, Zinknitrat oder eine Mischung davon umfasst.

7. Mundpflegezusammensetzung, wie in irgendeinem der vorhergehenden Ansprüche beansprucht, wobei die Orthophosphat-Quelle löslich ist und eine Löslichkeit von mindestens 0,1 Mol dm⁻³ aufweist.

8. Mundpflegezusammensetzung, wie im Anspruch 7 beansprucht, wobei das Orthophosphat ein oder mehrere Salze der Formel (I) umfasst:
M₍₃₋ₙ₎HₙPO₄ (I),
worin:
- M Kalium, Natrium, Ammonium oder eine Kombination davon ist,
- n 0, 1 oder 2 ist.

9. Mundpflegezusammensetzung, wie in irgendeinem vorhergehenden Anspruch beansprucht, wobei die Zusammensetzung ein Zahnputzmittel, vorzugsweise eine Zahnpasta, ist.

10. Mundpflegezusammensetzung, wie in irgendeinem der vorhergehenden Ansprüche beansprucht, wobei die Zusammensetzung ein Tensid umfasst.

11. Mundpflegezusammensetzung, wie in irgendeinem der vorhergehenden Ansprüche beansprucht, wobei die Gesamtmenge des Zinks in der Zusammensetzung von 0,01 bis 10 Gewichts-% der Zusammensetzung beträgt.

12. Mundpflegezusammensetzung, wie in irgendeinem der vorhergehenden Ansprüche beansprucht, wobei die Gesamtmenge des Orthophosphats (PO₄) in der Zusammensetzung von 0,05 bis 20 Gewichts-% der Zusammensetzung beträgt.

13. Mundpflegezusammensetzung, wie in irgendeinem der vorhergehenden Ansprüche beansprucht, zur Verwendung zum Verbessern der Mundhygiene eines Individuums, umfassend das Auftragen der Zusammensetzung auf mindestens eine Oberfläche der Zähne des Individuums.

## Revendications

1. Composition pour le soin oral comprenant :
a) une source de zinc ; et
b) une source d'orthophosphate ;
dans laquelle le rapport molaire de zinc à orthophosphate (Zn:PO₄) dans la composition est inférieur à 1:1 ;
dans laquelle si la composition pour le soin oral comprend une source de calcium le rapport molaire de zinc à calcium (Zn:Ca) est alors d'au moins 1:5 ;
dans laquelle le pH de la composition est supérieur à 6,0 ; dans laquelle le pH de la composition est mesuré lorsque 1 partie en masse de la composition est uniformément dispersée et/ou dissoute dans 20 parties en masse d'eau pure à 25°C ; et
dans laquelle la composition est :
I) une composition de mono-phase comprenant la source de zinc et la source d'orthophosphate, dans laquelle la composition comprend de l'eau dans une quantité de 0 à 5 % en masse de la composition ;
II) une composition de mono-phase hydrique dans laquelle la source de zinc est encapsulée, la source d'orthophosphate est encapsulée ou les deux ; ou
III) une composition de double-phase comprenant :
(i) une première phase comprenant la source de zinc ; et
(ii) une seconde phase comprenant la source d'orthophosphate.

2. Composition pour le soin oral selon la revendication 1, dans laquelle le rapport de Zn:PO₄ se trouve dans l'intervalle de moins de 1:1 à 1:20.

3. Composition pour le soin oral selon la revendication 1 ou la revendication 2, dans laquelle la composition est une composition de mono-phase comprenant de l'eau dans une quantité de 0 à 5 % en masse de la composition.

4. Composition pour le soin oral selon l'une quelconque des revendications précédentes, dans laquelle le pH de la composition est d'au moins 6,5.

5. Composition pour le soin oral selon l'une quelconque des revendications précédentes, dans laquelle la source de zinc est modérément soluble ayant une solubilité d'au moins 0,1 mmol dm⁻³ mais inférieure à 0,1 mol dm⁻³, de préférence une source de zinc modérément soluble comprenant du citrate de zinc, lactate de zinc ou un mélange de ceux-ci.

6. Composition pour le soin oral selon l'une quelconque des revendications 1 à 4, dans laquelle la source de zinc est soluble ayant une solubilité d'au moins 0,1 mol dm⁻³, de préférence une source de zinc soluble comprenant du chlorure de zinc, acétate de zinc, ascorbate de zinc, sulfate de zinc, nitrate de zinc, ou un mélange de ceux-ci.

7. Composition pour le soin oral selon l'une quelconque des revendications précédentes, dans laquelle la source d'orthophosphate est soluble ayant une solubilité d'au moins 0,1 mol dm⁻³.

8. Composition pour le soin oral selon la revendication 7, dans laquelle l'orthophosphate comprend un ou plusieurs sels de formule (I) :
M₍₃₋ₙ₎HₙPO₄ (I) ;
dans laquelle :
- M est le potassium, sodium, ammonium ou une combinaison de ceux-ci ;
- n est égal à 0, 1 ou 2.

9. Composition pour le soin oral selon l'une quelconque des revendications précédentes, dans laquelle la composition est un dentifrice, de préférence une pâte dentaire.

10. Composition pour le soin oral selon l'une quelconque des revendications précédentes, dans laquelle la composition comprend un tensioactif.

11. Composition pour le soin oral selon l'une quelconque des revendications précédentes, dans laquelle la quantité totale de zinc dans la composition est de 0,01 à 10 % en masse de la composition.

12. Composition pour le soin oral selon l'une quelconque des revendications précédentes, dans laquelle la quantité totale d'orthophosphate (PO₄) dans la composition est de 0,05 à 20 % en masse de la composition.

13. Composition pour le soin oral selon l'une quelconque des revendications précédentes pour une utilisation dans l'amélioration de l'hygiène orale chez un individu comprenant l'application de la composition sur au moins une surface des dents de l'individu.
